# EUROPEAN PATENT APPLICATION

(11) **EP 4 702 939 A1**
(43) Date of publication of application: **04.03.2026**
(21) Application number: 24196777.7
(22) Date of filing: 27.08.2024
(51) Int. Cl.: A61B 17/80

(54) **BONE PLATE**

(71) Applicant: PMU Innovations GmbH, 5020 Salzburg (AT)
(72) Inventor: Fierlbeck, Johann, 83435 Bad Reichenhall (DE)
(74) Representative: Vossius & Partner Patentanwälte Rechtsanwälte mbB

(57) **Abstract**

The present invention relates to a medical bone plate for the treatment of fractures of the distal femur with metaphyseal comminution, periprosthetic fractures, and/or interprosthetic fractures, the bone plate being non-custom fit and non-planar, the bone plate being twisted and having a proximal portion, a distal portion and an intermediate portion connecting the proximal portion with the distal portion, wherein:
a. the distal portion is configured to fit to the distal medial femoral condyle;
b. the proximal portion is configured to be aligned with the distal ventral section of the femoral shaft.

## Description

The present invention relates to a medical bone plate for the treatment of fractures, and in particular to a medical bone plate for the treatment of fractures of the distal femur with metaphyseal comminution, periprosthetic fractures, and/or interprosthetic fractures.

Distal Femur fractures need accurate reduction and fixation guaranteeing functional stability to provide early mobilization and to maintain alignment until achievement of bone union. Especially in geriatric patients, post-operative weight bearing restrictions are difficult and the need of immediate mobilization after surgery, to prevent further medical problems is obligate. The treatment of said fractures requires a surgical procedure in which fragments of the distal femur are connected by screwing a bone plate to the fragments. Lateral plates using fixed-angle locking screws provide good stability, especially in the fixation of the distal fragment. For many cases this represents the gold standard today. But with increasing age associated with osteoporosis and increased physical activity, loss of reduction, resulting in varus deformity, is the most reported complication after open reduction and internal fixation (ORIF). To counteract this, double-plate fixation techniques were introduced. Literature describes different double plating techniques to bring more stability to the fracture site. In general, the lateral bone plate will be supplemented by a medial plate to create a stable pillar construction. The medial plate is, in most cases, a standard fracture plate, which is manually pre-contoured, i.e. adapted to the individual patient's femur, by the surgeon before or during the surgery.

However, manual bending includes some drawbacks. For example, it is time consuming, relatively inaccurate (e.g. due to inconvenient equipment in the operating room), and strongly depends of the surgeon's expertise to obtain the proper plate-shape to fit the plate to the patient's bone. In addition, repeated bending of a bone plate, which is generally made from titanium, may result in fractures in the bone plate. Especially bending back can reduce the possible cyclic stress on a bone plate and lead to bone plate fracture.

Moreover, and even with adequately adapted state of the art bone plates, tilting of the distal fracture fragment may occur in the bone plate treated femur. The varus deformity can be controlled by the double plating technique.

It is an object of the present invention to provide an improved bone plate that results in an improved surgical procedure and an improved biomechanical stability in the treated femur.

The present invention provides a pre-shaped medical anatomical bone plate for the distal medial femur, which can be placed in an anatomically safe corridor. A left version and a right version of the bone plate may be provided for a left or right femur, respectively. Embodiments of the bone plate support minimally invasive plate osteosynthesis (MIPO) using a medial surgical approach. Embodiments of the bone plate counteract the physiological loading and prevent tilting of the distal fragment, thus allowing full weight bearing after surgery, in many cases even immediately after the surgery.

This technique is particularly beneficial for the following fracture types, which often require a double plating technique incorporating a medial bone plate: AO/OTA classification 33A3, 33C1, 33C2, and 33C3 of the distal femur.

Regarding supracondylar periprosthetic femoral fractures, the following types can be addressed in the classification according Su et al.:

| | |
|---|---|
| Type I: | Fracture proximal to the femoral knee component. |
| Type II: | Fracture originating at the proximal aspect of the femoral knee component and extending proximally. |
| Type III: | Fracture where any part of the fracture line is distal to the upper edge of the anterior flange of the femoral knee component. |

In the context of periprosthetic distal femur fractures, ensuring the stability of the prosthesis is critical. The Rorabeck classification system is employed to assess and categorize these fractures, including the condition of the patient's prosthesis. Specifically, for Rorabeck Type 1 and Type 2 fractures, where the prosthesis remains stable, the preferred treatment method is angle-stable plate osteosynthesis.

The bone plates of the present invention may be configured for a treatment of one or a combination of such fractures.

The terms "proximal" and "distal" are used herein according to the following commonly known definition, which uses the torso as the reference point: "Proximal" is closer to the torso than "distal".

Moreover, the following definitions of planes and axes apply herein:
Axis X: The axis X is defined in a coronal plane as the horizontal line that best fits the two most distal points of the medial and lateral femoral condyles, passing through the center of the knee joint.
Axis Y: The Axis Y is defined by looking at the distal femur in a sagittal plane. Starting from the sagittal plane the distal femur is oriented such that the medial and lateral condyles are aligned in a single projection. Then, the axis Y is defined as a tangential line to the distal femoral condyle and parallel to a transversal plane, passing through the center of the knee joint.

Intersection and Plane Formation:
Femur Condyle Plane (FCP): Axes X and Y intersect at the center of the knee joint (knee center point), forming the femur condyle plane (FCP).
Central Axis Z: The central axis Z is perpendicular to the femur condyle plane and passes through the knee center point.

In particular, the present invention relates to a medical bone plate for the treatment of fractures of the distal femur with metaphyseal comminution, periprosthetic fractures, and/or interprosthetic fractures, the bone plate being non-custom fit and non-planar, the bone plate being twisted and having a proximal portion, a distal portion and an intermediate portion connecting the proximal portion with the distal portion, wherein:
a. the distal portion is configured to fit to the distal medial femoral condyle;
b. the proximal portion is configured to be aligned with the distal ventral section of the femoral shaft.

"Twisted" may refer to a shape that is bent in more than a single plane.

"Non-custom fit" means that identical medical bone plates may be implanted into different patients without further adaptation to the respective patient. It means that the medical bone plate is a "one-fits-many" device. However, different versions for, e.g. different femur sizes, left and right femurs, etc., are contemplated.

"Non-planar" may refer to a geometry that when put on a planar surface does not contact that surface with its entire surface area oriented toward that surface. For example, a straight bone plate (i.e., a bone plate that is not bent in any direction) as known in the art, that is still to be custom fit to a patient during surgery, would be considered a planar bone plate (despite being a 3D object).

The distal portion may comprise a distal ventral portion configured to fit the ventral medial femoral condyle as a first branch and a distal dorsal portion configured to fit the medial femoral condyle dorsally of the medial collateral ligament as a second branch, wherein the distal portion preferably bifurcates into the distal ventral portion and the distal dorsal portion.

Additionally or alternatively, the distal dorsal portion of the bone plate may have at least one hole for a bone fixation element, i.e. the hole is configured to receive or accommodate the bone fixation element. Additionally or alternatively, the distal ventral portion of the bone plate may have at least one hole for a bone fixation element, i.e. the hole is configured to receive or accommodate the bone fixation element.

Additionally or alternatively, the distance, as measured in sagittal plane, between the at least one hole for the bone fixation element of the distal dorsal portion and the at least one hole for the bone fixation element of the distal ventral portion prevents a nutation movement in the sagittal plane at the medial condyle. In other words, the two holes for the bone fixation elements form a two point fixation option in sagittal plane which prevent the femoral condyle from tipping cranially because of the rotation moment acting on the femoral condyle. For example, said distance may be from 20mm to 30mm, preferably 27mm, as measured from center to center of the holes in sagittal plane. In case of more than one hole in at least one of the branches, the distance may refer to the distance between the distalmost hole in the distal dorsal portion and the distalmost hole in the distal ventral portion. Additionally or alternatively, In case of more than one hole in at least one of the branches, the distance may refer to the largest distance between any pair of holes having one hole in the distal dorsal portion and one hole in the distal ventral portion. Distances of holes may be measured from center to center.

In preferred embodiments including the branched distal portion, e.g. the distal dorsal portion and the distal ventral portion, the medical bone plate may provide for a particularly stable bone fragment fixation. Moreover, the shape may be advantageous in that a single shape may fit various femurs of different patients without the need for customization prior or during the surgery. Nevertheless, due to significant differences in femur sizes, the medical bone plate may be engineered in different sizes to fit femurs of patients of different sizes. For example, there may be a "small" version for small patients, a "medium" version for medium sized patients, and a "large" version for tall patients.

Additionally or alternatively, the proximal portion of the bone plate may have at least two holes, each of the holes being configured to receive or accommodate a bone fixation element. For example, the proximal portion of the bone plate may have two, three, four, five, six, or more such holes.

Additionally or alternatively, the intermediate portion may be configured to be free of bone fixation elements.

Additionally or alternatively, one or more of the holes of the proximal portion may be configured to accommodate bone fixation elements that have a first diameter, at least one hole of the distal portion may be configured to accommodate a bone fixation element that has a second diameter, and the first diameter differs from the second diameter, wherein the first diameter of the bone fixation element is preferably larger than the second diameter. At least one of the holes of the proximal portion may have a third diameter and at least one of the holes of the distal portion may have a fourth diameter. The third diameter and the fourth diameter may be equal. Nevertheless, said holes may accommodate bone fixation elements of different diameters, for example when the bone fixation elements have different diameters but comprise heads that are large enough for both hole diameters. Alternatively, the third diameter may differ from the fourth diameter. For example, the third diameter may be larger than the fourth diameter. One or some of the holes of the proximal portion may have a diameter that differs from the diameter of at least one of the holes of the distal portion, while one or some of the holes of the proximal portion may have the same diameter as at least one of the holes of the distal portion.

Additionally or alternatively, at least some of the holes, e.g. all holes, may be configured to accommodate fixed angle bone fixation elements. Additionally or alternatively, at least some of the holes, e.g. all holes, may be configured to accommodate variable-angle bone fixation elements.

Additionally or alternatively, the distal portion may have a Distal Portion Start Point (in short "Start DP") and a Distal Portion End Point (in short "End DP"). The proximal portion may have a Proximal Portion start Point (in short "Start PP") and a Proximal Portion End Point (in short "End PP").

Additionally or alternatively, the distal portion of the bone plate may have one or any combination of the following distances of centers of holes for bone fixation elements to the femur condyle plane as measured parallel to the Z axis:
- Start DP: 17 mm to 32 mm, preferably 23 mm;
- End DP: 39 mm to 54 mm, preferably 47 mm;
wherein Start DP refers to a most distal center of a hole for a bone fixation element in the distal portion, and End DP refers to a most proximal center of a hole for a bone fixation element of the distal portion.

Start DP and End DP may both be associated with the medial femoral condyle, i.e. the bone fixation elements through the corresponding holes may be configured to attach to the medial femoral condyle.

Additionally or alternatively, the proximal portion of the bone plate may have one or any combination of the following distances of centers of holes for bone fixation elements to the femur condyle plane as measured parallel to the Z axis:
- Start PP: 90 mm to 105 mm, preferably 97 mm;
- End PP: 160 mm to 250 mm, preferably 185 mm;
wherein Start PP refers to a most distal center of a hole for a bone fixation element of the proximal portion, and End PP refers to a most proximal center of a hole for a bone fixation element of the proximal portion.

Start PP and End PP may both be associated with the femoral shaft, i.e. the bone fixation elements through the corresponding holes may be configured to attach to the femoral shaft.

Additionally or alternatively, the distal end of the distal ventral portion may be used as a reference point for the distances of centers of holes for bone fixation elements as measured parallel to the Z axis. It is, however, preferred, that the distance of the Start DP, End DP, Start PP, and End PP to the distal end of the distal ventral portion take into account the possibility to shift the bone plate's position along the femur (i.e. in Z direction) to achieve the best possible fit in combination with the ranges for the positions of the holes for different bone sizes.

For example, the distal portion of the bone plate may have one or any combination of the following distances of centers of holes for bone fixation elements to the distal end of the distal ventral portion as measured parallel to the Z axis:
- Start DP: 5mm to 11mm preferably 8mm;
- End DP: 24mm to 36mm preferably 32mm;
wherein Start DP and End DP are defined as above.

Additionally or alternatively, the proximal portion of the bone plate may have one or any combination of the following distances of centers of holes for bone fixation elements to the distal end of the distal ventral portion as measured parallel to the Z axis:
- Start PP: 77mm to 92mm preferably 84mm;
- End PP: 129mm to 219mm preferably 154mm;
wherein Start PP and End PP are defined as above.

The three portions of the bone plate, proximal, distal, and intermediate portions, may have specific technical functions. The distal portion of the bone plate may be specifically engineered to stabilize the fracture using bone fixation elements, which may be effective during patient mobilization. The medial positioning of the plate on the medial femoral condyle may prevent cranial tipping of the fragment. By branching the bone plate into a distal ventral portion and a distal dorsal portion, an improved lever arm may be provided for the bone fixation elements to counteract rotational movements in the sagittal plane of the distal bone fragment attached to the bone plate. Biomechanical loads acting on the distal femur may be transferred via the bone fixation elements to the distal portion of the bone plate. It is favorable for the distal portion to have an, as far as possible, optimized anatomical fit to minimize irritation to the soft tissue and the medial collateral ligament.

The intermediate portion of the bone plate is responsible for transferring the loads from the distal portion to the proximal portion. Generally, the intermediate portion bridges the fracture zone. Its shape may conform to the anatomy of the medial epicondyle of the femur, including a twist from medial to ventral.

The proximal portion is engineered to achieve an anatomical fit on the distal ventral femur shaft, providing the necessary mechanical stability to transmit loads from the intermediate portion to the un-fractured part of the femur via bone fixation elements.

The present invention also relates to a set comprising the medical bone plate as described herein with at least two holes for a bone fixation element in the proximal portion and at least one hole for a bone fixation element in the distal portion, a bone fixation element for the proximal portion fitting at least one of the at least two proximal holes, and a bone fixation element for the distal portion fitting at least one of the at least one distal holes, wherein the bone fixation element for the proximal portion has a first diameter and the bone fixation element for the distal portion has a second diameter, and wherein the first diameter differs from the second diameter, wherein the first diameter is preferably larger than the second diameter.

The bone plate may be non-custom fit. The shape of the bone plate may be based on data of femurs from a plurality of CT-scans of anatomical specimen.. Thus, the bone plate according to the present invention may be configured to be a one-fits-many device, i.e. identical bone plates may be used with different patients without further customization.

A method for engineering a medical bone plate may be as follows:
The shape of a bone plate can be accomplished using a standard CAD system as follows: Anatomical landmarks are identified on the virtual bone model, ensuring they can be easily recognized despite anatomical variability. It is advantageous if these landmarks correspond to the position of the intended bone plate. In this instance, the femoral condyle plane and the axis Z serve as the base reference for all structural elements.

In the second step, the necessary anchoring points for the bone fixation elements are defined, the anchoring points being selected to stabilize specific fracture patterns with the bone plate. These anchoring points must be accessible via the surgical approach without compromising critical anatomical structures such as arteries, nerves, or ligaments. Here, the anchoring points are defined in the distal medial area of the femoral condyle, ventral and lateral to the medial collateral ligament. Additional anchoring points are identified on the distal ventral femoral shaft, proximal to the femoral medial epicondyle.

The anchoring points are then connected by a volume that conforms to the anatomical shape of the bone. Typically, a compromise is necessary to account for anatomical variance in the femurs from the plurality of CT-scans of anatomical specimen. In practice, various cuts are made through one or more bone models based on the base reference. These cuts mimic the eventual position of the bone plate on the bone and are used to determine guide curves. By manipulating these guide curves, it is possible to refine the best possible anatomical fit despite bone anatomical variance. The volume of the bone plate is subsequently created by assigning different or identical cross-sections along the guide curves.

The invention is further described with reference to the Figures, wherein
- Figure 1: shows an exemplary CT image of a right distal femur in sagittal plane (left image) and coronal plane (right image) to illustrate the axes X and Y spanning the femur condyle plane, and to illustrate the axis Z;
- Figure 2: shows a left side view of an embodiment of a left bone plate according to the invention i.e. a view along the X axis from medial towards lateral;
- Figure 3: shows a front view of the embodiment of Fig. 2 i.e. a view along the Y axis from ventral towards dorsal;
- Figure 4: shows a right side view of the embodiment of Fig. 2 i.e. a view along the X axis from lateral towards medial;
- Figure 5: shows a top view of the embodiment of Fig.2, i.e. a view along the Z axis from the proximal towards the distal end of the bone plate;
- Figure 6: shows a bottom view of the embodiment of Fig. 2, i.e. a view along the Z axis from the distal towards the proximal end of the bone plate;
- Figure 7: shows a perspective view of the embodiment of Fig. 2 with bone fixation elements; and
- Figure 8: shows the embodiment of Fig. 2 on a left distal femur.

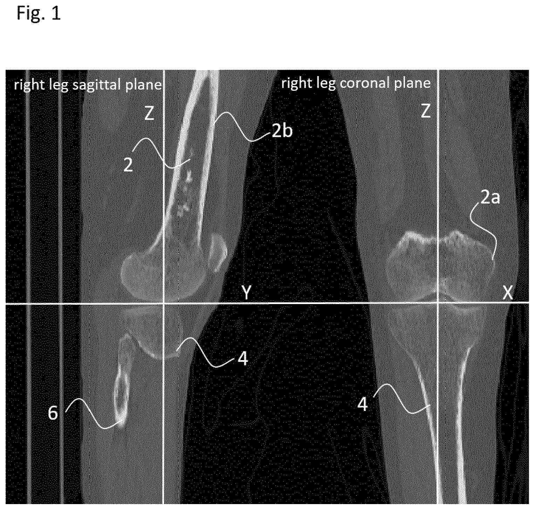

Figure 1 shows an exemplary CT image of a right distal femur 2 together with tibia 4 and fibula 6 in sagittal plane (left image) and coronal plane (right image) to illustrate the axes X and Y spanning the femur condyle plane (FCP), and to illustrate the axis Z. A definition of the axes and the FCP is provided above.

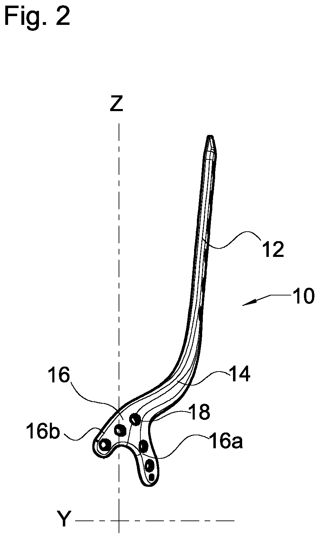

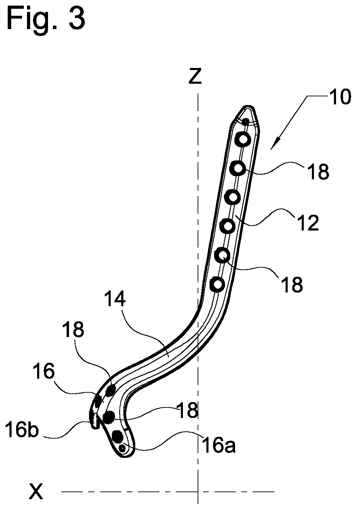

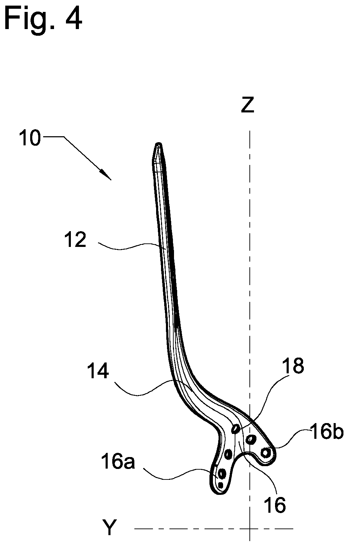

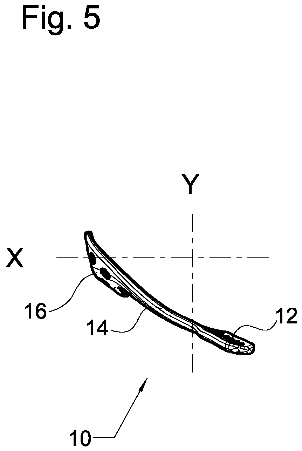

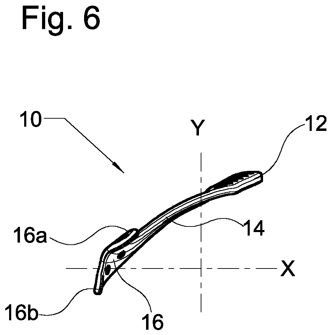

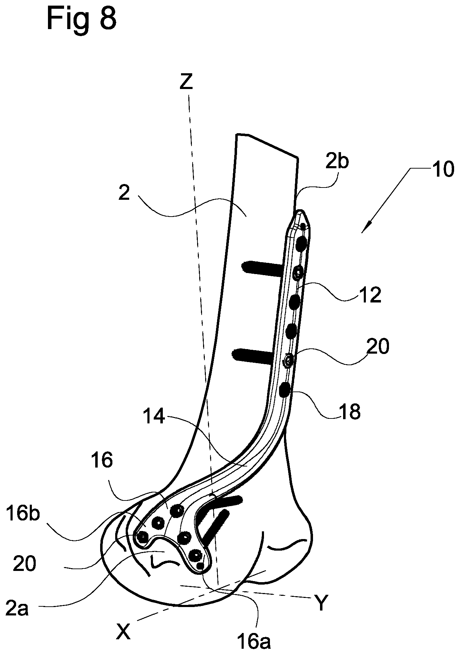

Figures 2-6 show a left side embodiment of a medical bone plate 10 (herein also just "bone plate") according to the invention in different views. Figure 8 shows the bone plate 10 in its intended position on a left distal femur 2. The bone plate 10 has a proximal portion 12, an intermediate portion 14, and a distal portion 16. As shown, the bone plate 10 may have two or more holes 18 for bone fixation elements 20. The holes 18 of the bone plate 10 enable fixation of the bone plate 10 to the distal femur 2 with bone fixation elements 20. For the sake of clarity, reference sign 18 is shown for exemplary holes only.

As can be best seen in Fig. 8, the bone plate 10 has a twisted shape. The distal portion 16 is configured to fit to the distal medial femoral condyle 2a. The proximal portion is configured to be aligned with the distal ventral section of the femoral shaft 2b of the femur 2.

The bone plate 10 may extend from its proximal end in a relatively straight line along the femoral shaft 2b and then make a twist from ventral to medial and in a distal direction to run along the distal medial femoral condyle 2a.

The distal portion 16 may comprise a distal ventral portion 16a configured to fit the ventral medial femoral condyle 2a as a first branch and a distal dorsal portion 16b configured to fit the medial femoral condyle 2a dorsally of the medial collateral ligament (not shown) as a second branch. Preferably, the distal portion 16 bifurcates into the distal ventral portion 16a and the distal dorsal portion 16b, i.e. other branches are not present in the distal portion 16.

As shown in the figures, the bone plate 10 may comprise at least one hole 18 in the distal portion 16 and at least one hole 18, preferably at least two holes 18, more preferably 4, 5, or 6 holes 18 in the proximal portion 12. Preferably, the distal dorsal portion 16a of the bone plate 10 has at least one hole 18 for a bone fixation element 20. Preferably, the distal ventral portion 16a of the bone plate 10 has at least one hole 18 for a bone fixation element 20. The distance of the at least one hole 18 in the distal ventral portion 16a to the at least one hole 18 in the distal dorsal portion, as measured center to center, may be in a range that prevents a nutation movement in the sagittal plane at the medial condyle 2a. The distance may be in a range from 20mm to 30mm, e.g., 27mm. In case of more than one hole 18 in the distal branches 16a, 16b, the distance may refer to the distance of the distalmost hole 18 of the distal ventral portion 16a to the distalmost hole 18 in the distal dorsal portion 16b. Additionally or alternatively, the distance may refer to the pair of holes 18 with the largest distance. As shown, the intermediate portion 14 may be free of any holes 18 for bone fixation elements 20.

The holes 18 for the bone fixation elements 20 may have different diameters, e.g. to accommodate bone fixation elements 20 of different diameters. For example, at least one hole 18, e.g. all holes 18, of the proximal portion 12 may have a first diameter, at least one hole 18, e.g. all holes 18, of the distal portion 16 may have a second diameter, and the first diameter may differ from the second diameter. For example, the first diameter may be larger than the second diameter. Additionally or alternatively, the diameter may vary among the holes 18 of the distal portion 16. Additionally or alternatively, the diameter may vary among the holes 18 of the proximal portion 12. Additionally or alternatively, the different diameters as specified for the holes 18 may apply to the respective bone fixation elements 20. The holes 18 may be configured to accommodate fixed angle bone fixation elements 20 or variable angle bone fixation elements 20. For example, the one or more holes 18 in the proximal portion 12 may be configured to accommodate fixed angle bone fixation elements 20, and/or the one or more holes in the distal portion 16 may be configured to accommodate variable angle bone fixation elements 20, or vice versa. It is also contemplated to have fixed and variable angle bone fixation elements 20 in the proximal portion 12, and/or to have fixed and variable angle bone fixation elements 20 in the distal portion 16. Any combination of fixed and variable angle bone fixation elements 20 in the proximal and distal portions 12, 16 is contemplated.

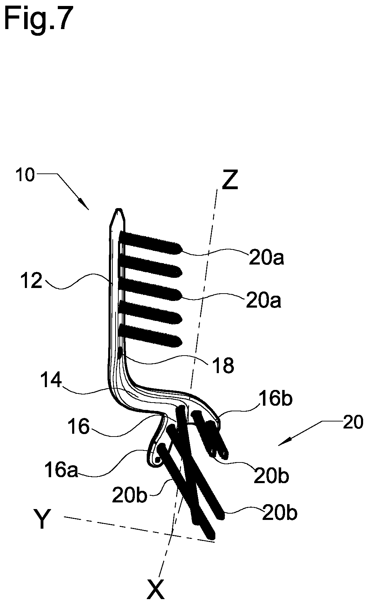

In Fig. 7, the bone plate 10 is shown with bone fixation elements 20 inserted into the bone plate 10. Bone fixation elements 20 may be, e.g., fixed-angle bone fixation elements (here: screws) and/or variable-angle bone fixation elements (here screws), as known in the art, and as described above. For the sake of clarity only exemplary reference signs 20, 20a and 20b (see below for description of 20a and 20b) are shown in Figs. 7 and 8.

The bone plate 10 may be made from any suitable material, preferably metal, preferably titanium or stainless steel. The bone plate 10 may be a single piece of material.

The distal portion 16 of the bone plate 10 may have one or any combination of the following distances of holes 18 for bone fixation elements 20 to the femur condyle plane as measured from the FCP to the centers of the holes 18 parallel to the Z axis:
- Start DP: 17 mm to 32 mm, preferably 23 mm;
- End DP: 39 mm to 54 mm, preferably 47 mm;
wherein Start DP and End DP are defined as specified above.

The proximal portion 12 of the bone plate 10 may have one or any combination of the following distances of holes 18 for bone fixation elements 20 to the femur condyle plane as measured from the FCP to the centers of the holes 18 parallel to the Z axis:
- Start PP: 90 mm to 105 mm, preferably 97 mm;
- End PP: 160 mm to 250 mm, preferably 185 mm;
wherein Start PP and End PP are defined as specified above.

Figure 7 shows an embodiment of a set according to the present invention. As already mentioned, Fig. 7 shows the medical bone plate 10 according to the present invention, as well as compatible bone fixation elements 20. The bone fixation elements 20 with reference sign 20a are inserted in the holes 18 of the proximal portion 12, and the bone fixation elements 20 with reference sign 20b are inserted in the holes 18 of the distal portion 16. One or more of the bone fixation elements 20a of the proximal portion 12 may have a third diameter, and one or more of the bone fixation elements 20b of the distal portion may have a fourth diameter that differs from the third diameter. The third diameter is preferably larger than the fourth diameter.

### List of Reference Signs:

- 2: distal femur
- 2a: medial condyle
- 2b: femoral shaft
- 4: tibia
- 6: fibula
- 10: medical bone plate
- 12: proximal portion
- 14: intermediate portion
- 16: distal portion
- 16a: distal ventral portion
- 16b: distal dorsal portion
- 18: hole
- 20: bone fixation element
- 20a: bone fixation element in proximal portion
- 20b: bone fixation element in distal portion

## Claims

1. A medical bone plate for the treatment of fractures of the distal femur with metaphyseal comminution, periprosthetic fractures, and/or interprosthetic fractures, the bone plate being non-custom fit and non-planar, the bone plate being twisted and having a proximal portion, a distal portion and an intermediate portion connecting the proximal portion with the distal portion, wherein:
a. the distal portion is configured to fit to the distal medial femoral condyle;
b. the proximal portion is configured to be aligned with the distal ventral section of the femoral shaft.

2. The medical bone plate of claim 1, wherein the distal portion comprises a distal ventral portion configured to fit the ventral medial femoral condyle as a first branch and a distal dorsal portion configured to fit the medial femoral condyle dorsally of the medial collateral ligament as a second branch, wherein the distal portion preferably bifurcates into the distal ventral portion and the distal dorsal portion.

3. The medical bone plate of claim 2, wherein the distal dorsal portion of the bone plate has at least one hole configured to accommodate a bone fixation element.

4. The medical bone plate of any of claim 2 or 3, wherein the distal ventral portion of the bone plate has at least one hole configured to accommodate a bone fixation element.

5. The medical bone plate of claim 4 as depending on claim 3, wherein the distance between the at least one hole for the bone fixation element of the distal dorsal portion and the at least one hole for the bone fixation element of the distal ventral portion, as measured from center to center in sagittal plane, prevents a nutation movement in the sagittal plane at the medial condyle.

6. The medical bone plate of any of the preceding claims, wherein the proximal portion of the bone plate has at least two holes, each of the holes being configured to accommodate a bone fixation element.

7. The medical bone plate of any of the preceding claims, wherein the intermediate portion is configured to be free of bone fixation elements.

8. The medical bone plate of any of claims 6-7 as depending on claims 3-5, wherein one or more of the holes of the proximal portion are configured to accommodate bone fixation elements that have a first diameter, at least one hole of the distal portion is configured to accommodate a bone fixation element that has a second diameter, and the first diameter differs from the second diameter, wherein the first diameter is preferably larger than the second diameter.

9. The medical bone plate of any of claims 3-8, wherein at least some of the holes, e.g. all holes, are configured to accommodate fixed angle bone fixation elements.

10. The medical bone plate of any of claims 3-9, wherein at least some of the holes, e.g. all holes, are configured to accommodate variable-angle bone fixation elements.

11. The medical bone plate of any of the preceding claims, wherein a cross section of the intermediate portion of the bone plate is different from a cross section of the proximal portion and from a cross section of the distal portion.

12. The medical bone plate of any of the preceding claims, wherein the distal portion of the bone plate has one or any combination of the following distances of centers of holes for bone fixation elements to the femur condyle plane as measured parallel to the Z axis:
- Start DP: 17 mm to 32 mm, preferably 23 mm;
- End DP: 39 mm to 54 mm, preferably 47 mm;
wherein Start DP refers to a most distal center of a hole for a bone fixation element in the distal portion, and End DP refers to a most proximal center of a hole for a bone fixation element of the distal portion.

13. The medical bone plate of any of the preceding claims, wherein the proximal portion of the bone plate has one or any combination of the following distances of centers of holes for bone fixation elements to the femur condyle plane as measured parallel to the Z axis:
- Start PP: 90 mm to 105 mm, preferably 97 mm;
- End PP: 160 mm to 250 mm, preferably 185 mm;
wherein Start PP refers to a most distal center of a hole for a bone fixation element of the proximal portion, and End PP refers to a most proximal center of a hole for a bone fixation element of the proximal portion.

14. A set comprising the medical bone plate of any of claims 6-13 as depending from any one of claims 3-5, a bone fixation element for the proximal portion fitting at least one of the at least two proximal holes, and a bone fixation element for the distal portion fitting at least one of the at least one distal holes, wherein the bone fixation element for the proximal portion has a third diameter and the bone fixation element for the distal portion has a fourth diameter, and wherein the third diameter differs from the fourth diameter, wherein the third diameter is preferably larger than the fourth diameter.
